# DEMANDE DE BREVET EUROPEEN

(11) **EP 1 516 871 A1**
(43) Date de publication de la demande: **23.03.2005**
(21) Numéro de dépôt: 04292211.2
(22) Date de dépôt: 15.09.2004
(51) Int. Cl.: C07D 209/60, A61K 31/4025, A61P 25/00, A61P 15/00

(54) **Dérivés de benzoindoline avec une activité adrénergique**

(30) Priorité: 16.09.2003 FR 0310828
(71) Demandeur: Les Laboratoires Servier, 92415 Courbevoie Cedex (FR)
(72) Inventeur: Lavielle, Gilbert, 78170 La Celle Saint Cloud (FR); Muller, Olivier, 95300 Pontoise (FR); Millan, Mark, 78230 Le Pecq (FR); Gobert, Alain, 92500 Rueil-Malmaison (FR); Di Cara, Benjamin, 13260 Cassis (FR)

(57) **Abrégé**

Composés de formule (I) : 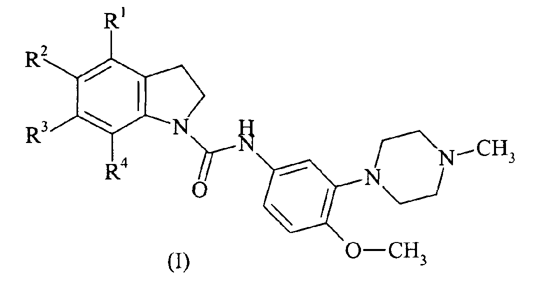 dans laquelle :
R¹, R², R³ et R⁴ sont tels que définis dans la description,
et leurs sels d'addition à un acide ou à une base pharmaceutiquement acceptable,
Médicaments.

## Description

La présente invention, sélection du brevet EP-1170288, a pour objet de nouveaux dérivés de benzoindoline leur procédé de préparation et les compositions pharmaceutiques qui les contiennent.

Le cortex frontal joue un rôle essentiel dans les processus qui contrôlent les fonctions altérées dans les désordres psychiatriques. En particulier, il est maintenant admis que la perturbation de la transmission noradrénergique, dopaminergique et cholinergique est fortement impliquée dans l'étiologie de ces différents troubles. Par exemple dans le cas de la dépression, l'activité de ces neuromédiateurs est diminuée au niveau des régions corticolimbiques.

Parmi les différentes classes d'auto- et hétérorécepteurs de monoamines impliqués dans les mécanismes de régulation, les récepteurs α₂-AR (Adréno Récepteurs) et 5-HT_{2C} ont montré une importance capitale. Ces deux sous-types réceptoriels agissent dans le même sens en inhibant la transmission dopaminergique et adrénergique. D'une part un contrôle inhibiteur est exercé par les récepteurs α₂-AR, sur les neurones dopaminergiques, noradrénergiques et cholinergiques [*The Journal of Pharmacology and Experimental Therapeutics,* 270, 958 *(1994) ; European Journal of Neuroscience,* 12, 1079-1095 (2000), *The Journal of Pharmacology and Experimental Therapeutics,* 305, 338-346 (2003)], et d'autre part, les récepteurs 5-HT_{2C} exercent un contrôle inhibiteur sur la transmission dopaminergique et noradrénergique [*Neuropharmacology*, 36, 609 (1997)].

Des composés se liant à l'une ou l'autre de ces classes réceptorielles ont montré leur potentiel, par le passé, dans le traitement de plusieurs pathologies.

Ainsi le rôle bénéfique de composés antagonistes α₂-AR a été étudié dans le traitement des troubles cognitifs [*Psychopharmacology*, 123, 239-249 (1996); *Journal of Psychopharmacology*, 6, 3, 376-381 (1992); *Neuroscience Letters,* 142, 36-40 (1992) *; European Journal of Pharmacology*, 277, 113-116 (1995)], de la maladie de Parkinson [*CNS Drugs,* 10, 189 (1998)], des troubles de la libido et des dysfonctionnements sexuels [*Journal of Pharmacology*, 11, 72 (1997) ; *Urology,* 49, 3, 441-444 (1997); *International Journal of* *Impotence Research,* 12, S70-S74 (2000); *International Journal of Impotence Research,* 12, Suppl 1, S75-S80 (2000); *World Journal of Urology,* 19, 51-56 (2001)], de la shizophrénie *[British Journal of Pharmacology*, 124, 1550-1556 (1998)], et de la dépression [*European Journal of Neuroscience,* 12, 1079-95 (2000); *The Journal of Pharmacology and Experimental Therapeutics,* 277 (2), 852-60 (1997); *Naunyn-Schmiedeberg's Archiv. Pharmacol*., 355 (1), 20-9 (1997)].

De la même façon des composés antagonistes des récepteurs 5HT_{2C} ont montré leur utilité dans le traitement des dysfonctionnements sexuels [*Life Sciences,* 45, 1263-1270 (1989) ; *Pharmacological Biochemistry and Behavior,* 39, 605-612 (1991); *Psychopharmacology,* 119, 291-294 (1995); *Neuroendocrinology*, 76, 28-34 (2002)] de la maladie de Parkinson [*Drug News Perspect,* 12 (8), 477-483 (1999)], mais aussi de la dépression [*Prog. Neuro-Psychopharmacol. Biol. Psychiat.,* 18, 563-574 (1994) ; *British Journal of Psychiatry*, 171, 444-448 (1997) ; *Neuropharmacology,* 39, 1222-1236 (2000)], de l'anxiété [*British Journal of Pharmacology,* 117, 427 (1996)], de la schizophrénie [*Neuroscience Letter,* 181, 65 (1996); *The Journal of Pharmacology and Experimental Therapeutics,* 295, 1, 226-232 (2000); *Molecular Psychiatry,* 6, 373-379 (2001); *The Journal of Neuroscience,* 21, 19, 7781-7787 (2001); *Pharmacology, Biochemistry and Behavior,* 71, 745-756 (2002) ; *Psychopharmacology*, 162, 55-62 (2002)], des troubles du comportement impulsif [*The Journal of Pharmacology and Experimental Therapeutics,* 298 (2), 581-591 (2001) ; *Brain research*, 835, 104-112 (1999)], et des troubles du sommeil [*Psychopharmacology,* 96, 395-399 (1988) ; *Neuropsychopharmacology*, 21, 455-466 (1999) ; *Biological Psychiatry,* 47, 468-470 (2000) ; *Pharmacology, Biochemistry and Behavior,* 71, 599-605 (2002)].

Des composés possédant un caractère double d'antagonistes α₂-AR et 5-HT_{2C} peuvent être d'une grande utilité pour les cliniciens, afin d'obtenir, avec l'administration d'un même composé, une action considérablement renforcée, par un effet de synergie, au niveau de la restauration de la neurotransmission. Ce type de composé présente de plus un avantage considérable par rapport à l'administration de deux produits différents.

Les composés de l'invention présentent une structure originale benzoindoline qui leur confère ce caractère d'antagonistes doubles α₂-AR/5-HT_{2C} et sont donc utiles dans le traitement de la dépression, de l'anxiété, de la schizophrénie, de la maladie de Parkinson, des troubles cognitifs, des troubles de la libido et des dysfonctionnements sexuels, des troubles du sommeil, et des troubles du comportement impulsif.

La présente invention concerne de nouveaux dérivés de benzoindoline qui constituent une sélection des composés décrits de la demande EP-1170288. Ces composés sont nouveaux et se différencient de ceux décrits et exemplifiés dans la demande EP-1170288 non seulement par l'absence d'atome d'halogène sur l'indoline, mais surtout par la présence d'un groupement benzo fusionné au groupement indoline.

De manière surprenante, l'introduction de ce groupement confère aux composés de l'invention des activités pharmacologiques nettement supérieures à celles du composé de l'art antérieur le plus proche structurellement (exemple 11 de la demande EP-1170288 : la 6-chloro-5-fluoro-*N*-[4-méthoxy-3-(4-méthyl-1-pipérazinyl)phényl]-1-indoline-carboxamide).

Ainsi, l'utilisation du radical benzoindoline a permis d'améliorer remarquablement les propriétés pharmacologiques des composés de l'invention.

Plus spécifiquement, la présente invention concerne les composés de formule (I) : 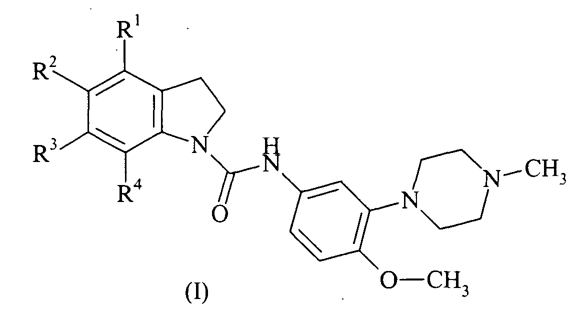 dans laquelle :
R¹, R² forment ensemble un cycle benzo éventuellement substitué par un atome d'halogène ou un groupement alkyle, alkoxy, cyano, nitro, hydroxy, amino, alkylamino, dialkylamino ou trifluorométhyle, et R³, R⁴ représentent un atome d'hydrogène,
ou
R¹, R⁴ représentent un atome d'hydrogène et R², R³ forment ensemble un cycle benzo éventuellement substitué par un atome d'halogène ou un groupement alkyle, alkoxy, cyano, nitro, hydroxy, amino, alkylamino, dialkylamino ou trifluorométhyle,
ou alors
R¹, R² représentent un atome d'hydrogène et, R³, R⁴ forment ensemble un cycle benzo éventuellement substitué par un atome d'halogène ou un groupement alkyle, alkoxy, cyano, nitro, hydroxy, amino, alkylamino, dialkylamino ou trifluorométhyle,
leurs énantiomères, diastéréoisomères et leurs sels d'addition à un acide ou à une base pharmaceutiquement acceptable,
étant entendu que :
- le terme alkyle désigne une chaîne hydrocarbonée, linéaire ou ramifiée contenant de 1 à 6 atomes de carbone,
- le terme alkoxy désigne un groupement alkyle-oxy, linéaire ou ramifié, contenant de 1 à 6 atomes de carbone.

Parmi les acides pharmaceutiquement acceptables, on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer l'hydroxyde de sodium, l'hydroxyde de potassium, la triéthylamine, la tertbutylamine, etc...

Un aspect avantageux de l'invention concerne les composés pour lesquels R¹, R² forment ensemble un cycle benzo éventuellement substitué par un atome d'halogène ou un groupement alkyle, alkoxy, cyano, nitro, hydroxy, amino, alkylamino, dialkylamino ou trifluorométhyle, et R³, R⁴ représentent un atome d'hydrogène.

Parmi les composés préférés de l'invention, on peut citer plus particulièrement la *N*-[4-méthoxy-3-(4-méthyl-1-pipérazinyl)phényl]-1,2-dihydro-3*H*-benzo[*e*]indole-3-carboxamide.

La présente invention concerne également le procédé de préparation des composés de formule (I) caractérisé en ce que l'on utilise comme produit de départ un dérivé benzoindole de formule (II) : 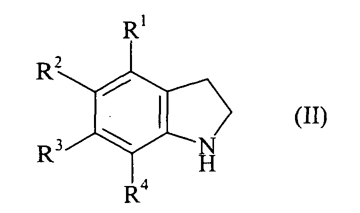 dans laquelle R¹, R², R³ et R⁴ sont tels que définis dans la formule (I),
qui se condense avec un dérivé de formule (III) : 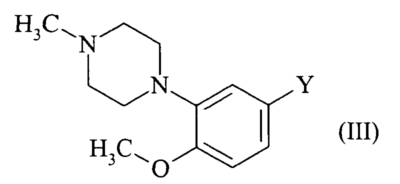 dans laquelle Y représente un groupement -N=C=O ou -C(O)-N₃,
pour conduire au composé de formule (I),
- qui peut être le cas échéant purifié selon une technique classique de purification,
- dont les isomères (diastéréoisomères et énantiomères) sont séparés si nécessaire par une technique classique de séparation,
- que l'on transforme, si on le souhaite, en ses sels d'addition à un acide ou à une base pharmaceutiquement acceptable,
étant entendu que l'indoline de formule (II) est préparée selon des modes opératoires connus, par exemple à partir de dérivé nitronaphtylacétonitrile correspondant.

La présente invention a également pour objet les compositions pharmaceutiques renfermant comme principe actif au moins un composé de formule (I), seul ou en combinaison avec un ou plusieurs excipients ou véhicules inertes non toxiques, pharmaceutiquement acceptable.

Parmi les compositions pharmaceutiques selon l'invention, on pourra citer plus particulièrement celles qui conviennent pour l'administration orale, parentérale, nasale, transdermique, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,05 et 500 mg pour un traitement en 1 à 3 prises par 24 heures.

Les exemples qui suivent illustrent l'invention et ne la limitent en aucune façon.

Les structures des composés décrits ont été confirmées par les techniques spectroscopiques et spectrométriques usuelles.

Les produits de départ utilisés sont des produits connus ou préparés selon des modes opératoires connus.

### PREPARATION 1 : 2,3-Dihydro-1H-benzo[e]indole

### Stade A : (2-Nitro-1-naphtyl)acétonitrile

Préparer une solution de 53,5 g (0,477 mole) de tertiobutylate de potassium dans 400 ml de diméthylformamide. Refroidir cette solution à -10°C et y additionner en 1 h environ une solution de 40 g de 4-chlorophénoxyacétonitrile (0,24 mole) et 37 g de 2-nitronaphtalène (0,213 mole) dans 200 ml de diméthylformamide. Après 2 heures à -5°C, verser-le tout sur 4 1 d'eau contenant 1 l d'acide chlorhydrique concentré et extraire la phase aqueuse avec 3 x 500 ml de dichlorométhane. Laver la phase organique avec 300 ml d'eau, la sécher sur sulfate de magnésium, filtrer puis évaporer le solvant.
On obtient 65 g de produit.
Recristalliser ces 65 g dans un mélange cyclohexane/acétate d'éthyle : 50/50 : v/v.

### Stade B : 3H-Benzo[e]indole

Hydrogéner à température ambiante sous 4 bars d'hydrogène 33 g de (2-nitro-1-naphtyl)acétonitrile (0,155 mole) dissous dans 630 ml d'éthanol à 10 % d'eau et 6,3 ml d'acide acétique pur : utiliser 19 g de palladium/charbon à 10 %. Après cessation de l'absorption, filtrer le catalyseur, concentrer le solvant sous vide puis reprendre le résidu dans 250 ml de dichlorométhane, laver la phase organique avec 100 ml de solution 0,1 N d'hydroxyde de potassium, puis sécher la phase organique sur sulfate de magnésium, filtrer et concentrer.
Le résidu est purifié par chromatographie sur silice éluant cyclohexane/acétate d'éthyle : 80/20 : v/v.

### Stade C : 2,3-Dihydro-1H-benzo[e]indole

10 g (0,06 mole) du composé préparé au stade précédent, sont dissous dans 50 ml de tétrahydrofurane. Ajouter à cette solution, à 0°C, 120 ml de complexe borane/THF en solution 1 M dans le tétrahydrofurane, puis 120 ml d'acide trifluoroacétique. Après 30 minutes, ajouter à 0°C, 6 ml d'eau, laisser agiter 15 minutes puis concentrer le tout à sec. Le résidu est repris dans 200 ml de dichlorométhane et lavé avec 200 ml de soude 1 N. La phase organique est séchée sur sulfate de magnésium, filtrée et concentrée.

### PREPARATION 2 : 2,3-Dihydro-1H-benzo[f]indole

Le protocole expérimental pour réduire la 1*H*-benzo[*f*]indole est identique à celui de la Préparation 1, Stade C. La synthèse de la 1*H*-benzo[*f*]indole de départ a été décrite dans la littérature *[Tetrahedron,* **49,** 33, 7353 (1993); *Heterocycles,* 24, 7, 1845, (1986)].

### PREPARATION 3 : 2,3-Dihydro-1H-benzo[e]indole-6-carbonitrile

### Stade A : N-(5-Cyano-2-naphtyl)acétamide

A 25 g de *N*-(5,6,7,8-tétrahydronaphtalèn-2-yl)acétamide refroidie à 0°C additionner successivement 70 ml de cyanure de triméthylsilyl pur puis 30 g de dichlorodicyanoquinone dans 70 ml de dichlorométhane. Après 3 heures à température ambiante ajouter à nouveau une solution de 60 g de dichlorodicyanoquinone dans 140 ml de dichlorométhane. Laisser agiter 12 heures à 20°C puis porter 8 heures à 60°C.
Après neutralisation par une solution saturée d'hydrogénocarbonate de sodium, la phase organique est décantée et lavée par de l'eau. Le résidu obtenu après concentration est purifié par chromatographie sur gel de silice en utilisant comme éluant un mélange cyclohexane/acétate d'éthyle : 80/20 :v/v.

### Stade B : N-(1-Bromo-5-cyano-2-naphtyl)acétamide

A une solution refroidie à -5°C de 50 g (0.238 mole) du produit synthétisé au stade précédent dans 500 ml de dichlorométhane et 25 ml de pyridine, additionner 39,8 g de brome dissous dans 200 ml de dichlorométhane. Laisser ensuite agiter fortement pendant 4 heures à température ambiante, puis diluer avec 500 ml de dichlorométhane, laver la phase organique avec 2 fois 300 ml d'eau, sécher et concentrer. Le résidu est recristallisé dans un mélange dichlorométhane/méthanol : 50/50 : v/v.

### Stade C : 6-Amino-5-bromo-1-naphtonitrile

Chauffer 6 heures à 80°C un mélange de 12,5 g (0,043 mole) du produit synthétisé au stade précédent, 3,6 g d'hydroxyde de sodium, 195 ml de méthanol et 65 ml d'eau.
Après évaporation du méthanol la phase aqueuse est extraite deux fois par du dichlorométhane. Celui-ci est ensuite séché et évaporé. Le résidu est cristallisé dans un mélange dichlorométhane-méthanol.

### Stade D : 1-Bromo-5-cyano-2-naphtylcarbamate d'éthyle

Additionner à 0°C 19 ml de chloroformiate d'éthyle à une solution de 33 g (0,133 mole) du produit synthétisé au stade précédent dans 200 ml de pyridine. Après 1 heure à 5°C, évaporer le solvant, reprendre le résidu par 500 ml de dichlorométhane, laver la phase organique par 3 fois 100 ml d'acide chlorhydrique 0,1N, puis par 200 ml d'une solution d'hydrogénocarbonate de sodium à 10 % et enfin une fois à l'eau. Le résidu obtenu par évaporation est recristallisé dans un mélange dichlorométhane-méthanol.

### Stade E : 5-Cyano-1-[(triméthylsilyl)éthynyl]-2-naphtylcarbamate d'éthyle

Dans un réacteur en acier mélanger 14,1 g (0,044 mole) du produit synthétisé au stade précédent, 11 ml de triméthylsilylacétylène, 13ml de triéthylamine, 670 mg d'iodure cuivreux et 1,54 g de dichloro bis(triphénylphosphine)palladium. Fermer ensuite le réacteur et porter le mélange réactionnel à 80°C pendant 4 heures. Diluer avec 200 ml de dichlorométhane et 100 ml d'eau, filtrer le tout, décanter la phase organique, la sécher et évaporer le solvant sous vide. Le résidu obtenu est purifié par chromatographie sur gel de silice en utilisant comme éluant un mélange cyclohexane/acétate d'éthyle : 90/10 : v/v puis cristallisation dans le même solvant.

### Stade F : 3H-benzo[e]indole-6-carbonitrile

A une solution de 2,74 g de sodium dans 280 ml d'éthanol sec ajouter 10 g (0,0297 mole) du produit synthétisé au stade précédent et porter le tout une heure à reflux. Après évaporation du solvant, le résidu est repris dans 200 ml de dichlorométhane et la phase organique est lavée par 200 ml d'eau. Après évaporation le résidu est purifié par chromatographie sur gel de silice en utilisant comme éluant un mélange cyclohexane/acétate d'éthyle : 80/20 : v/v.

### Stade G : 2,3-dihydro-1H-benzo[e]indole-6-carbonitrile

Le protocole expérimental pour réduire le 3*H*-benzo[*e*]indole-6-carbonitrile est identique à celui de la Préparation 1, Stade C.

### PREPARATION 4 : 7-Méthoxy-2,3-dihydro-1H-benzo[e]indole

### Stade A : 6-Méthoxy-3,4-dihydro-1(2H)-naphtalénone oxime

Solubiliser 100 g (0,57 mol) de 6-méthoxy-1-tétralone dans 2,5 l d'un mélange éthanol/eau : 80/20. Ensuite sont additionnés à température ambiante 85 g (1,04 mol) d'acétate de sodium et 43 g (0,62 mol) de chlorhydrate d'hydroxylamine. Porter au reflux 4 heures la suspension. Diluer le milieu par 5 l d'eau et extraire à l'éther éthylique, laver à l'eau, sécher sur sulfate de magnésium, filtrer. Après évaporation du solvant on obtient 99 g d'un solide beige.

### Stade B : 2-Amino-6-méthoxy-3,4-dihydro-1(2H)-naphtalénone

Solubiliser 50 g (0,26 mol) ) du produit synthétisé au stade précédent dans 185 ml de pyridine, puis additionner à température ambiante 54,9 g (0,29 mol) de chlorure de paratoluènesulfonyle. Après 24 heures verser sur de la glace puis filtrer le précipité. Reprendre le précipité dans du dichlorométhane et laver à l'eau, sécher la phase organique sur sulfate de magnésium, filtrer. Après évaporation du solvant on obtient 89 g d'un solide jaune (produit intermédiaire 1).

Ajouter 7,48 g (0,32 mol) de sodium dans un mélange toluène/éthanol : 720/148 ml. Après dissolution, diluer par 940 ml de toluène et additionner rapidement 117 g (0,34 mol) du produit intermédiaire 1. Après 24 heures à température ambiante, filtrer le paratoluènesulfonate de sodium rincer au toluène, puis verser la solution organique sur une solution d'acide chlorhydrique 10 % (1,1 l). Décanter, extraire une fois à l'eau puis évaporer la phase aqueuse. Reprendre le résidu dans de l'éthanol puis filtrer le précipité. On obtient 46,5 g d'un solide beige sous forme chlorhydrate.

### Stade C: N-Ethyl-N'-(6-méthoxy-1-oxo-1,2,3,4-tétrahydro-2-naphtalényl)urée

Verser 4,77 g (0,044 mol) de chloroformiate d'éthyle à 0°C sur 5 g (0,022 mol) de 2-amino-6-méthoxy-3,4-dihydro-2*H*-naphtalèn-1-one en solution dans la pyrydine. Après deux heures à température ambiante concentrer la pyridine, reprendre par du dichlorométhane puis laver la phase organique par une solution d'acide chlorhydrique 0,1N puis une solution saturée d'hydrogénocarbonate de sodium et de l'eau, sécher sur sulfate de magnésium, filtrer, évaporer le solvant. 5,48 g d'un solide orange sont obtenus.

### Stade D : N-Ethyl-N'-(6-méthoxy-1,2,3,4-tétrahydro-2-naphtalènyl)urée

Hydrogéner à 60°C sous pression atmosphérique 98 g (0,37 mole) du produit précédemment préparé au Stade C, dissout dans 1,51 d'éthanol avec 10 g de palladium/charbon à 5 %. Après cessation de l'absorption, filtrer le catalyseur, concentrer le solvant sous vide. On obtient 88,2 g d'une huile.

### Stade E : N-Ethyl-N'-(6-méthoxy-2-naphtyl)urée

Solubiliser 7,42 g (0,0298 mol) du produit synthétisé au stade précédent, dans 100 ml de toluène. Ajouter 13,51 g (0,0595 mol) de dichlorodicyanoquinone et porter au reflux 30 minutes. Filtrer à température ambiante le précipité, rincer au toluène puis évaporer le solvant. Le résidu est purifié par chromatographie sur gel de silice en utilisant comme éluant du dichlorométhane pur. On obtient 4 g d'un solide gris

### Stade F : 6-Méthoxy-2-naphtylamine

Solubiliser 3,5 g du produit synthétisé au stade précédent, dans 65 ml d'éthanol ensuite ajouter une solution d'hydroxyde de potassium dans 65 ml d'eau. Après 4 heures de reflux filtrer à température ambiante le précipiter formé. Reprendre le brut par du dichlorométhane et laver à l'eau jusqu'à neutralité, sécher sur sulfate de magnésium, filtrer le précipité puis évaporer le solvant. On obtient 2,02 g d'un solide orange.

### Stade G : 1-Iodo-6-méthoxy-2-naphtylamine

28,2 g (0,16 mol) du produit synthétisé au stade précédent, sont dissous dans un mélange dichlorométhane/méthanol : 1500/620 ml. Ajouter à cette solution à température ambiante 56,7 g (0,16 mol) de benzyltriméthylammonium dichloroiodate et 21,2 g (0,212 mol) de carbonate de calcium. Après 30 minutes filtrer l'insoluble puis reprendre la phase organique par une solution de bisulfite de sodium à 10 % et extraire à l'éther éthylique. Sécher sur sulfate de magnésium, filtrer puis évaporer. Le résidu est purifié par chromatographie sur gel de silice en utilisant comme éluant un mélange cyclohéxane/acétate d'éthyle : 70/30 : v/v.

### Stade H: 1-Iodo-6-méthoxy-2-naphtylcarbamate d'éthyle

La transformation du 1-iodo-6-méthoxy-2-naphthylamine s'effectue en appliquant la méthode décrite au Stade C.

### Stade I : 6-Méthoxy-1-[(triméthylsilyl)éthynyl]-2-naphtylcarbamate d'éthyle

La transformation 1-iodo-6-méthoxy-2-naphtylcarbamate d'éthyle s'effectue en appliquant la méthode décrite dans la Préparation 3, Stade E.

### Stade J : 7-Méthoxy-3H-benzo[e]indole

La transformation du composé du stade précédent s'effectue en appliquant la méthode décrite dans la Préparation 3, Stade F.

### Stade K : 7-Méthoxy-2,3-dihydro-1H-benzo[e]indole

Le protocole expérimental pour réduire le 7-méthoxy-3*H*-benzo[*e*]indole est identique à celui de la Préparation 1, Stade C.

### PREPARATION 5 : 4-Méthoxy-3-(4-méthyl-1-pipérazinyl)benzoyl azide

Additionner à température ambiante une solution de 5,3 g (0,025 mole) de dichlorophosphate de phényle dans 100 ml de dichlorométhane à une solution de 5 g (0,02 mole) d'acide 4-méthoxy-3-(4-méthyl-pipérazin-1-yl)benzoïque [*J*. *Med. Chem*., 37 (15), 2255 (1994)] et 3,25 g (0,05 mole) d'azoture de sodium dans 4,05 ml de pyridine. Après 12 heures d'agitation, laver la phase organique avec 100 ml d'eau, décanter la phase organique, la sécher sur sulfate de magnésium et évaporer le solvant sous vide à 30 °C.

### EXEMPLE 1 : Chlorhydrate du N-[4-méthoxy-3-(4-méthyl-1-pipérazinyl)phényl]-1,2-dihydro-3H-benzo[e]indole-3-carboxamide

Porter à reflux une solution de 29 g (0,105 mole) du composé synthétisé dans la Préparation 5 dans 400 ml de toluène, puis refroidir à 20 °C, ajouter 100 ml de dichlorométhane puis une solution de 18 g (0,105 mole) du produit obtenu dans la Préparation 1. Après 24 heures d'agitation à 20 °C, évaporer le solvant et purifier le résidu par chromatographie sur silice en utilisant comme éluant un mélange dichlorométhane/méthanol/ammoniaque : 95/5/0,5 : v/v/v.
La base est ensuite salifiée par l'acide chlorhydrique en solution éthanolique.
*Point de fusion : 176-178 °C*.

### EXEMPLE 2 : Chlorhydrate du N-[4-méthoxy-3-(4-méthyl-1-pipérazinyl)phényl]-2,3-dihydro-1H-benzo[f]indole-1-carboxamide

Le protocole expérimental est identique à celui de l'Exemple 1 en partant du produit de la Préparation 2.

### EXEMPLE 3 : Chlorhydrate de 6-cyano-N-[4-méthoxy-3-(4-méthyl-1-pipérazinyl)-phényl]-1,2-dihydro-3H-benzo[e]-indole-3-carboxamide

Le protocole expérimental est identique à celui de l'Exemple 1 en partant du produit de la Préparation 3.

### EXEMPLE 4 : Chlorhydrate de 7-méthoxy-N-[4-méthoxy-3-(4-méthyl-1-pipérazinyl)-phényl]-1,2-dihydro-3H-benzo[e]-indole-3-carboxamide

Le protocole expérimental est identique à celui de l'Exemple 1 en partant du produit de la Préparation 4.

### ETUDES PHARMACOLOGIQUES

### EXEMPLE A : Détermination de l'affinité pour les récepteurs α₂ adrénergiques chez le rat

L'affinité a été déterminée par des expériences de compétition avec le [³H]-RX 821,002. Les membranes sont préparées à partir de cortex cérébral de rat et incubées en triple avec 0.4 nM de [³H]-RX 821,002 et le composé à tester dans un volume final de 1,0 ml, pendant 60 minutes à 22°C. Le tampon d'incubation contient 50 nM de TRIS-HCl (pH 7,5), 1 mM de EDTA et 100 µM de GppNHp. La fixation non spécifique est déterminée avec 10 µM de phentolamine.

*Analyse de données* : A la fin de l'incubation, le milieu d'incubation est filtré au travers de filtres WHATMAN GF/B imprégnés avec 0,1 % de polyéthylénimine et lavé trois fois avec 5 ml de tampon refroidi. La radioactivité retenue sur les filtres est détermine par comptage du liquide de scintillation. Les isothermes de binding sont analysées par régression non-linéaire.

*Résultats :* Les composés de l'invention montrent une activité antagoniste spécifique des récepteurs α₂-adrénergiques avec, par exemple pour le composé de l'Exemple 1 un pKi de 7,4, alors que pour le comparateur, (exemple 11 de la demande EP-1170288) la 6-chloro-5-fluoro-*N*-[4-méthoxy-3-(4-méthyl-1-pipérazinyl)phény]-1-indolinecarboxamide, le pKi est de 6,4.

### EXEMPLE B : Détermination de l'affinité pour les récepteurs 5-HT_{2C}

L'affinité des composés de l'invention a été déterminée par des expériences de compétition en présence de [³H]-mésulergine, dans un tampon d'incubation Hepes 20 mM, EDTA 2mM, acide ascorbique 0.1 % (pH=7,7), à 22°C. La constante de dissociation K_{D} de la [³H]-mésulergine est de 0,54 mM. La fraction non spécifique est définie en présence de 1 µM de miansérine, cette dernière étant également le produit de référence pour chaque expérience.
A la fin de la période d'incubation le milieu est filtré au travers de filtres GF/B-Unifilter pré-traités au PEI (0,1 %), suivi de trois lavages avec le tampon d'incubation. La radioactivité retenue sur les filtres est déterminée par comptage du liquide de scintillation. Les isothermes de binding sont analysées par régression non-linéaire pour déterminer les valeurs d'IC₅₀. Elles sont converties en pKᵢ (où Kᵢ est la constante de dissociation).

Il apparaît que les composés de l'invention possèdent une haute affinité pour les récepteurs 5-HT_{2c}, par exemple le composé de l'Exemple 1 possède un pKi de 8,2 alors que le comparateur, (exemple 11 de la demande EP-1170288) la 6-chloro-5-fluoro-*N*-[4-méthoxy-3-(4-méthyl-1-pipérazinyl)phényl]-1-indolinecarboxamide, a un pKi de 7,4.

### EXEMPLE C : Mesure de neurotransmetteurs dans le cortex frontal de rats

### Dialyse.

La chirurgie est réalisée sous anesthésie au pentobarbital (60 mg/kg, i.p.). Les rats males Wistar, 200-220 g (Iffa Credo, l'Arbresle, France), sont placés dans un appareil stéréotaxique de Kopf et un guide de canule (CMAMicrodialyse AB, Stockholm, Suède) implanté dans le cortex frontal aux coordonnées (en mm): antéropostérieur: + 2.2, latéral: ± 0.6, déviation ventrale: - 0.2. Les rats, mis en cage individuelle, récupèrent de l'anesthésie pendant 5 jours. Le jour de la dialyse, une sonde en cuprophan CMA/11 (4 mm de long, 0.24 mm de diam. ext.) est introduite dans le guide et perfusée à 1 µl/min. par une solution de NaCl: 147.2mM; KCl: 4mM; CaCl₂: 2.3mM, amenée grâce à un tampon phosphate à pH 7.3. Deux heures après l'implantation, des échantillons de dialysât sont collectés toutes les 20 min. pendant 4 hrs. Trois échantillons basaux sont collectés avant l'administration de la drogue.

### Chromatographie.

La noradrénaline (NA) et la dopamine (DA) sont mesurées de la manière suivante: 20 µl d'échantillon de dialysât sont dilués avec 20µl de phase mobile (NaH₂PO₄: 75 mM, EDTA: 20 µM, sodium décanesulphonate: 1 mM, méthanol: 17.5 %, triéthylamine 0.01 %, pH: 5.70) et 33 µl sont analysés par HPLC en utilisant pour séparation, une colonne phase inverse (hypersil C 18, 150 x 4.6 mm, taille des particules, 5 µm) thermostatée à 43°C et pour quantification, un détecteur coulométrique (ESA5014, Coulochem II, ESA, Chelmstord, USA). Le potentiel de la première électrode est de - 90 mV (réduction) et de la seconde de + 280 mV (oxydation). Le débit de la phase mobile est de 2 ml/min. La limite de sensibilité pour la NA et la DA est de 0.2 pg.

L'acétylcholine (ACh) est mesurée et quantifiée en absence d'inhibiteur de l'acétylcholine estérase (AChE). 20 µl d'échantillon de dialysât sont collectés sur 10µl d'acide acétique 0.01%. Des aliquots de 20µl sont analysés par HPLC. La phase mobile est composée de Na₂HPO₄: 50 mM et de proclin: 0.5% (BAS, Congleton, UK), pH 8.2. La phase stationnaire se compose d'une colonne échangeuse de cations (Sepstik, 530 x 1.0 mm, taille des particules, 10 µm, BAS), d'une pre-column (réacteur enzymatique choline oxydase/catalase, 55 x 1 mm, BAS) et d'une post-column (réacteur enzymatique choline oxydase/AChE, 50 x 1 mm, BAS). Le système est maintenu à 35°C. La quantification est réalisée grâce à un détecteur ampérométrique (LC-4B, BAS). Sur l'électrode de travail en carbone vitreux (MF2098, BAS) est déposé une couche de polymer peroxydase-redox. Le potentiel de cette électrode est de + 100 mV par rapport à l'électrode de référence Ag/AgCl. Le débit de la phase mobile est de 0.14 ml/min. La limite de sensibilité pour l'ACh est de 0.1 pg.

A titre d'exemple le composé de l'Exemple 1 (10.0 mg/kg, s.c.) induit une augmentation importante des concentrations extracellulaires en NA, DA et ACh dans les dialysas recueillis dans le cortex frontal des rats vigiles (Table 1). Le comparateur, (exemple 11 de la demande EP-1170288) la 6-chloro-5-fluoro-*N*-[4-méthoxy-3-(4-méthyl-1-pipérazinyl)-phényl]-1-indolinecarboxamide, (10.0 mg/kg, s.c.), induit une augmentation très modeste des concentrations extracellulaires en NA et DA et aucune modification de la concentration extracellulaire d'ACh (Table 1). Les niveaux de NA, DA et ACh sont exprimées comme le pourcentage de l'aire sous la courbe (% ASC) ± E.S.M. des temps 20 min. à 120 min. après l'administration de la drogue. Les effets du composé de l'Exemple 1 sont comparés (ANOVA) à ceux obtenus chez les animaux traités par le solvant ou par la 6-chloro-5-fluoro-*N*-[4-méthoxy-3-(4-méthyl-1-pipérazinyl)phényl]-1-indoline-carboxamide.

**Table 1**

| %ASC ± E.S.M. | Solvant | Composé de l'Exemple 1 | Comparateur (Exemple 11-EP-1170288) |
|---|---|---|---|
| NA | + 18.2 ± 5.0 | + 105.4 ± 7.3 ^{a}^{,}^{b} | + 39.8 ± 6.7 |
| DA | + 4.4 ± 2.5 | + 52.9 ± 6.6 ^{a} | + 19.5 ± 4.9 |
| ACh | + 16.5 ± 5.5 | + 107.7 ± 13.5 ^{a}^{,}^{b} | + 15.1 ± 12.3 |

| | | | |
|---|---|---|---|
| ^{a}, P < 0.005 versus solvant et | | | |
| ^{b}, P < 0.05 versus le 6-chloro-5-fluoro-*N*-[4-méthoxy-3-(4-méthyl-1-pipérazinyl)phényl]-1-indolinecarboxamide. | | | |

### EXEMPLE D : Composition pharmaceutique

| **Formule de préparation pour 1000 comprimés dosés à 10 mg** | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 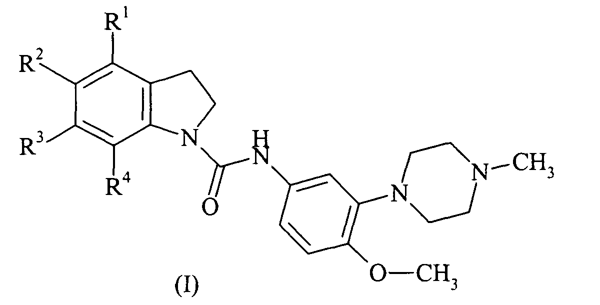 dans laquelle :
R¹, R² forment ensemble un cycle benzo éventuellement substitué par un atome d'halogène
ou un groupement alkyle, alkoxy, cyano, nitro, hydroxy, amino, alkylamino, dialkylamino
ou trifluorométhyle, et R³, R⁴ représentent un atome d'hydrogène,
ou
R¹, R⁴ représentent un atome d'hydrogène et R², R³ forment ensemble un cycle benzo éventuellement substitué par un atome d'halogène ou un groupement alkyle, alkoxy, cyano, nitro, hydroxy, amino, alkylamino, dialkylamino ou trifluorométhyle,
ou alors
R¹, R² représentent un atome d'hydrogène et, R³, R⁴ forment ensemble un cycle benzo éventuellement substitué par un atome d'halogène ou un groupement alkyle, alkoxy, cyano, nitro, hydroxy, amino, alkylamino, dialkylamino ou trifluorométhyle,
leurs énantiomères, diastéréoisomères et leurs sels d'addition à un acide ou à une base pharmaceutiquement acceptable,
étant entendu que :
- le terme alkyle désigne une chaîne hydrocarbonée, linéaire ou ramifiée contenant de 1 à 6 atomes de carbone,
- le terme alkoxy désigne un groupement alkyle-oxy, linéaire ou ramifié, contenant de 1 à 6 atomes de carbone.

2. Composés de formule (I), selon la revendication 1 pour lesquels R¹, R² forment ensemble un cycle benzo éventuellement substitué par un atome d'halogène ou un groupement alkyle, alkoxy, cyano, nitro, hydroxy, amino, alkylamino, dialkylamino ou trifluorométhyle, et R³, R⁴ représentent un atome d'hydrogène, leurs énantiomères, diastéréoisomères et leurs sels d'addition à un acide ou à une base pharmaceutiquement acceptable.

3. Composés de formule (I), selon une quelconques des revendications 1 ou 2 qui est la *N*-[4-méthoxy-3-(4-méthyl-1-pipérazinyl)phényl]-1,2-dihydro-3*H*-benzo[*e*]indole-3-carboxamide et ses sels d'addition à un acide ou à une base pharmaceutiquement acceptable.

4. Procédé de préparation d'un composé de formule (I), selon la revendication 1, **caractérisé en ce que** nous utilisions comme produit de départ un dérivé benzoindole de formule (II) : 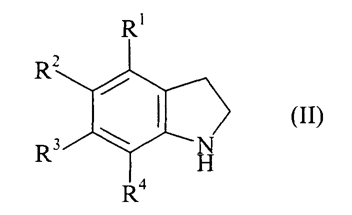 dans laquelle R¹, R², R³ et R⁴ sont tels que définis dans la formule (I),
qui se condense avec un dérivé de formule (III) : 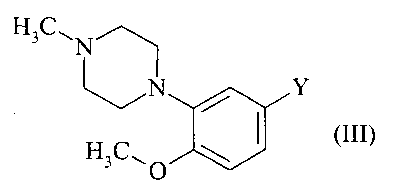 dans laquelle Y représente un groupement -N=C=O ou -C(O)-N₃,
pour conduire au composé de formule (I),
- qui peut être le cas échéant purifié selon une technique classique de purification,
- dont les isomères (diastéréoisomères et énantiomères) sont séparés si nécessaire par une technique classique de séparation,
que l'on transforme, si on le souhaite, en ses sels d'addition à un acide ou à une base pharmaceutiquement acceptable.

5. Compositions pharmaceutiques contenant comme principe actif au moins un composé selon l'une quelconque des revendications 1 à 3, seul ou en combinaison avec un ou plusieurs excipients ou véhicules inertes non toxiques, pharmaceutiquement acceptables.

6. Compositions pharmaceutiques selon la revendication 5 contenant au moins un principe actif selon l'une quelconque des revendications 1 à 3 utiles pour la fabrication de médicaments antagonistes double α₂-AR/5-HT_{2C}.

7. Compositions pharmaceutiques selon la revendication 5 contenant au moins un principe actif selon l'une quelconque des revendications 1 à 3 utiles pour la fabrication de médicaments utiles dans le traitement de la dépression, de l'anxiété, des désordres impulsifs, de la schizophrénie, de la maladie de Parkinson, des troubles cognitifs, des troubles de la libido et des dysfonctionnements sexuels, et des troubles du sommeil.
